# EUROPEAN PATENT APPLICATION

(11) **EP 1 093 829 A1**
(43) Date of publication of application: **25.04.2001**
(21) Application number: 00309275.6
(22) Date of filing: 20.10.2000
(51) Int. Cl.: A61N 1/39

(54) **Implantable medical device having a low voltage capacitor**

(30) Priority: 21.10.1999 US 422525
(71) Applicant: WILSON GREATBATCH LTD., Clarence New York 14031 (US)
(72) Inventor: Muffoletto, Barry C, Alden, New York 14004 (US); Shah, Ashish, East Amherst, New York 14051 (US)
(74) Representative: Bradley, Josephine Mary

(57) **Abstract**

The present invention relates to an implantable device. The implantable device has a low-voltage battery (10), a low-voltage capacitor (32), a high-voltage capacitor (12), and a charging circuit (16). The low-voltage capacitor (32) receives energy from the low-voltage battery (10). The high-voltage capacitor (12) receives energy from the low-voltage battery (10) and low-voltage capacitor (32), which are in parallel circuitry with each other and the high-voltage capacitor (32), through the charging circuit (16). And the charging circuit (16) converts the energy from the low-voltage battery (10) and the low-voltage capacitor (32) to a voltage typically greater than 500V stored in the high-voltage capacitor (12).

## Description

### BACKGROUND OF THE INVENTION

### 1. Field of the Invention

The present invention generally relates to reducing the size of an implantable battery unit, in particular for a defibrillator.

### 2. Prior Art

It is well known that cardiac defibrillators and pacemakers are commonly designed to be implanted within a human patient. Such cardiac defibrillators include an electrical energy storage component as part of a power supply designed to provide repeated burst discharges of several joules of electrical energy. Cardiac pacemakers include similar storage components designed to supply lower energy bursts but much more frequently. Both devices therefore require energy components of large capacity in order to reduce the number of occasions on which the device must be explanted to renew its energy storage component. It is therefore advantageous that the energy storage component is compact and capable of large energy storage. It is also advantageous if the energy storage component can be configured to the shape of the overall device, which is typically a flat, disc-shaped configuration to facilitate implantation subcutaneously in the patient. It is well known that aluminum electrolytic capacitors have some properties that are suited for this purpose.

A capacitor of this type conventionally includes an etched aluminum foil anode, an aluminum foil or film cathode, and an interposed Kraft paper or a fabric gauze separator impregnated with a solvent-based liquid electrolyte. Typically, the electrolytic or ion-producing component of the electrolyte is a salt that is dissolved in the solvent. The electrolyte thus provides ionic electrical conductivity from the cathode to an oxide layer that is typically formed on the aluminum anode and that functions as a dielectric layer between the anode and the cathode.

Allegedly, this type of capacitor has problems with the solvent-based liquid. To correct this problem, numerous inventors have patented other types of capacitors. Some of these capacitors are fully disclosed in U.S. Patent Numbers 4,942,501, 5,146,391, 5,153,820, 5,661,629. These and other designs can be utilized in the present invention.

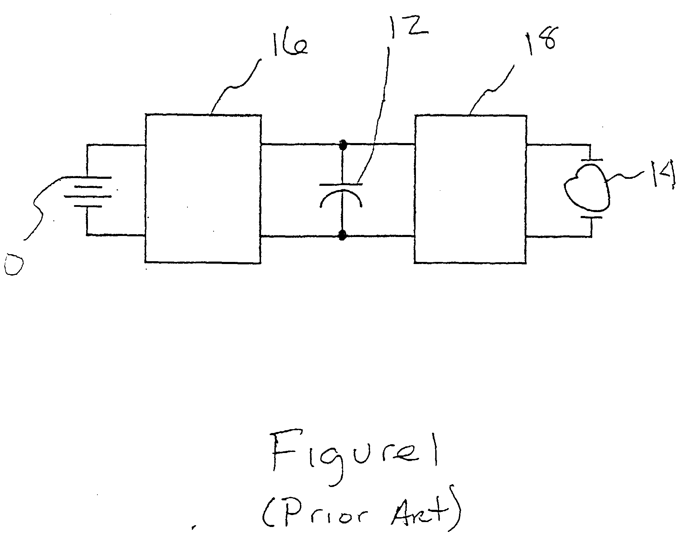

A defibrillator device requires high power circuitry. The conventional circuitry of such a device is illustrated in Figure 1. Figure 1 illustrates a battery unit 10, a high-voltage capacitor 12, and a heart 14, within a charging circuitry 16, and an output switching circuitry 18. The charging circuitry 16 must convert the energy from the battery 10, usually a low voltage like 3V if one battery or 6V if two batteries, to the much higher voltage, about 750 V, through the high-voltage charging section 16, charging capacitor 12. After the high voltage energy is concentrated in the high-voltage capacitor 12, output switching circuitry 18 transfers the energy to the heart 14. This circuitry 16, 18 should perform this function with minimal wasted energy, since implantable batteries and capacitors have limited energy capacity. Examples of the circuitry 16, 18 are illustrated, and not limited to, those described in Chapter 13 of Implantable Cardioverter Defibrillator Therapy - The Engineering-Clinical Interface, edited by M. Kroll and M. Lehmann, 1996, which is incorporated herein by reference.

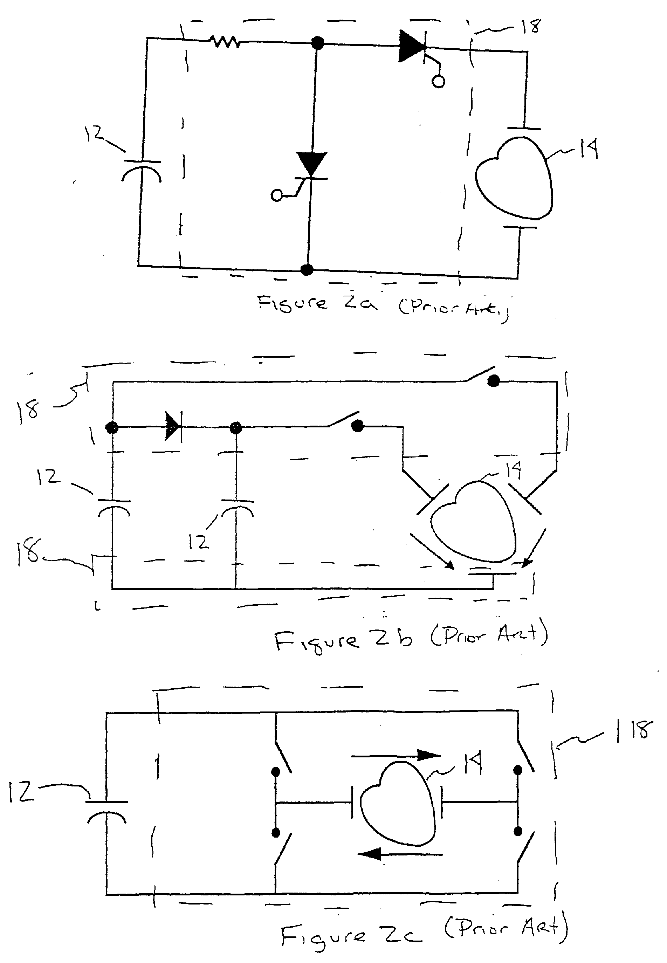

The time course of energy delivery is also critical. Only a few voltage waveforms that can be generated by implantable circuitry 18 are also currently known to be useful for internal defibrillation of the heart. The circuitry 18 is the monophasic truncated exponential (Figure 2a), the sequential monophasic truncated exponential (Figure 2b), or the biphasic truncated exponential (Figure 2c), all of which are fully described in the previously referenced Chapter 13 of Implantable Cardioverter Defibrillator Therapy - The Engineering-Clinical Interface, edited by M. Kroll and M. Lehmann, 1996. Each of these circuits 18 uses at least one high voltage capacitor. The sequential monophasis truncated exponential circuit 18 (Figure 2b) uses two capacitors, not in parallel, which are both high-voltage capacitors. None of the circuits 18 and 16 discloses a low voltage capacitor which is also in parallel with a high voltage capacitor and a battery.

### SUMMARY OF THE INVENTION

The present invention relates to an implantable device. The implantable device has a low-voltage battery, a low-voltage capacitor, a high-voltage capacitor, and a charging circuit. The low-voltage capacitor receives energy from the low-voltage battery. The high-voltage capacitor receives energy from the low-voltage battery and low-voltage capacitor, which are in parallel circuitry with each other and the high-voltage capacitor, through the charging circuit. And the charging circuit converts the energy from the low-voltage battery and the low-voltage capacitor to a voltage typically greater than 500 V stored in the high-voltage capacitor.

These and other aspects of the present invention will become more apparent to those skilled in the art by reference to the following description and to the appended drawings.

### BRIEF DESCRIPTION OF THE DRAWINGS

Figure 1 is a general electronic schematic of prior art implantable devices;
Figures 2a-2c are general electronic schematics of various prior art embodiments of the output switching circuitry; and
Figure 3 is an electrical schematic of the present invention.

### DETAILED DESCRIPTION OF THE PREFERRED EMBODIMENTS

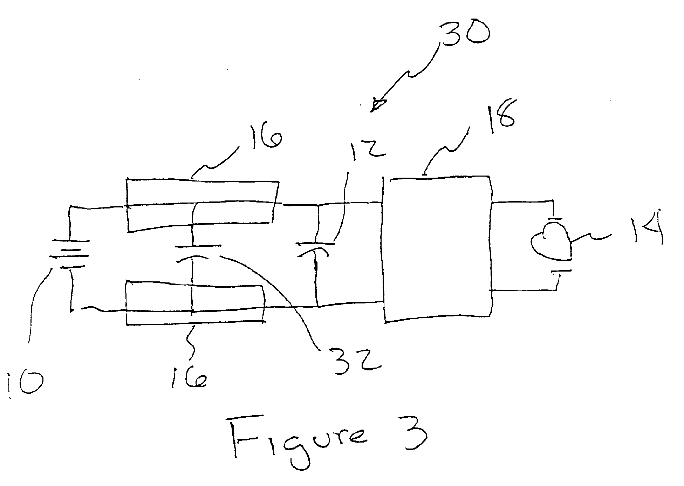

Referring now to the present invention, Figure 3 shows an embodiment of the present circuitry of an implantable medical device 30. The implantable medical device 30 has a battery 10, a low-voltage capacitor 32, a high-voltage capacitor 12, a charging circuitry 16, and an output switching circuitry 18. The present invention is similar to other implantable medical devices, except it has a low-voltage capacitor 32 placed in parallel with the battery 10 and the high-voltage capacitor 12.

The low-voltage capacitor 12 is any capacitor having an ability to store energy at less voltage than a high-voltage capacitor 12, which typically stores energy at more than 500 V. The low-voltage capacitor 32 augments the battery 10 when the battery recharges the high-voltage capacitor 12. With the low voltage capacitor 32 augmenting the battery 10, the time it takes to recharge the high-voltage capacitor 12 decreases.

Since the low voltage capacitor 32 augments the battery 10 during the high current drain period when the high voltage capacitor 12 is charged, the recharge time is decreased, and the battery 10 can (1) be smaller, (2) generate a lower voltage, and (3) be smaller and generate a lower voltage. A smaller battery means the overall size of the. device 30 can be smaller. Smaller is an attribute that is extremely desirable for an implantable device. Accordingly, the low-voltage capacitor 32 provides a multiple of advantages that were not previously disclosed in the prior art.

The charging circuitry 16 and the output switching circuitry 18 can contain a plurality of conventional circuitry elements, such as diodes and switches like FETs, MOSFETs, BJT, IGBT, inverter, and SCR. The exact circuitry 16, 18 can be any type of configuration. The configuration depends on the desired waveform, and desired voltage. However, the charging circuitry must transmit the energy from the battery 10 and low-voltage capacitor 32 to the high-voltage capacitor 12, and the output switching circuitry 18 must transmit the energy from the high-voltage capacitor 12 to the heart 14 of a user.

The battery 10 can be any conventional battery used within an implantable device.

The implantable medical device 30 can be stored in packaging, for example, a kit. When the device 30 is to be used, a person who works in a medical environment will remove the packaging and that person or another will surgically insert the device 30 into a patient, a human or another animal type.

It is appreciated that various modifications to the inventive concepts described herein may be apparent to those skilled in the art without departing from the spirit and scope of the present invention defined by the hereinafter appended claims.

## Claims

1. An implantable medical device, comprising:
a low-voltage battery;
a low-voltage capacitor receiving energy from the low-voltage battery, and storing the energy;
a high-voltage capacitor receiving energy from the low-voltage battery and low-voltage capacitor, each of which are in parallel circuitry with each other and the high-voltage capacitor, through a charging circuit; and
wherein the charging circuit converts the energy from the low-voltage battery and the low-voltage capacitor to a voltage typically greater than about 500 V stored in the high-voltage capacitor.

2. The device of claim 1 further comprising an output switching circuitry which transfers the energy from the high-voltage capacitor to a user's heart.

3. The device of claim 1 wherein the low-voltage capacitor and the battery transmit energy to the high-voltage capacitor to decrease the re-charging time of the high-voltage capacitor.

4. A method of making an implantable medical device comprising the steps of:
positioning a low-voltage battery, a low-voltage capacitor receiving energy from the low-voltage battery and storing the energy, and a high-voltage capacitor receiving energy from the low-voltage battery and low-voltage capacitor, in a parallel circuitry configuration within a charging circuit which converts the energy from the low-voltage battery and the low-voltage capacitor to a voltage typically greater than about 500 V stored in the high-voltage capacitor; and
packaging the device.

5. The method of claim 4 further comprising the step of connecting an output switching circuitry, which transfers the energy from the high-voltage capacitor to a user's heart.

6. The method of claim 4 wherein the low-voltage capacitor and the battery transmit energy to the high-voltage capacitor to decrease the re-charging time of the high-voltage capacitor.
